(19) Europäisches Patentamt
European Patent Office
Office européen des brevets

(11) Veröffentlichungsnummer: **0 546 374 A2**

(12) **EUROPÄISCHE PATENTANMELDUNG**

(21) Anmeldenummer: **92120001.0**

(22) Anmeldetag: **25.11.92**

(51) Int. Cl.5: **C07C 63/04**, C07C 63/70, C07C 65/21, C07C 51/255

(30) Priorität: **10.12.91 DE 4140645**

(43) Veröffentlichungstag der Anmeldung: **16.06.93 Patentblatt 93/24**

(84) Benannte Vertragsstaaten: **BE CH DE FR GB LI NL**

(71) Anmelder: **BASF Aktiengesellschaft**
**Carl-Bosch-Strasse 38**
**W-6700 Ludwigshafen(DE)**

(72) Erfinder: **Northemann, Andreas, Dr.**
**Rheinfeldstrasse 24**
**W-6700 Ludwigshafen(DE)**
Erfinder: **Fischer, Martin, Dr.**
**Elbinger Weg 1**
**W-6700 Ludwigshafen(DE)**

(54) **Verfahren zur Herstellung von ortho,ortho-disubstituierten Benzoesäuren.**

(57) Verfahren zur Herstellung von o,o-disubstituierten Benzoesäuren der allgemeinen Formel I

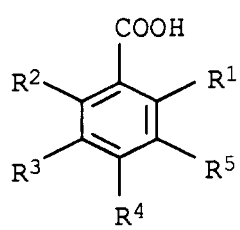

(I),

in der

$R^1,R^2,R^3,R^4,R^5$ $C_1$- bis $C_8$-Alkyl, $C_1$- bis $C_8$-Alkoxy, $C_3$- bis $C_8$-Cycloalkyl, $C_4$- bis $C_{20}$-Alkyl-cycloalkyl, Halogen, Aryl, $C_7$- bis $C_{20}$-Aralkyl und $C_7$- bis $C_{20}$-Alkylaryl

$R^3,R^4,R^5$ zusätzlich Wasserstoff,

bedeuten, indem man o,o-disubstituierte Aldehyde der allgemeinen Formel II

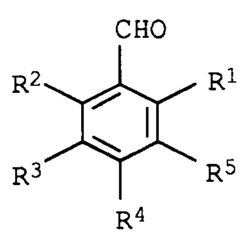

(II),

in der die Reste $R^1$, $R^2$, $R^3$, $R^4$ und $R^5$ die oben genannten Bedeutungen besitzen, durch Oxidation mit Sauerstoff oder Sauerstoff enthaltenden Gasen in Gegenwart von katalytischen Mengen eines Übergangsmetallsalzes bei Temperaturen von 50 bis 180 °C und gegebenenfalls eines Promotors umsetzt.

EP 0 546 374 A2

Die vorliegende Erfindung betrifft ein Verfahren zur Herstellung von o,o-disubstituierten Benzoesäuren durch Oxidation von o,o-disubstituierten Aldehyden mit Sauerstoff oder Sauerstoff enthaltenden Gasen in Gegenwart katalytischer Mengen eines geeigneten Übergangsmetallsalzes bei erhöhten Temperaturen.

Es ist bekannt, daß sich substituierte Benzaldehyde mit den verschiedensten Oxidationsmitteln, wie z.B. Mangandioxid, Chromoxiden, Kaliumpermanganat, zu den entsprechenden Benzoesäuren oxidieren lassen (Houben-Weyl, Methoden der Organischen Chemie, 4. Auflage, Band IV/1a, Seite 135 bis 137). Diese Verfahren sind jedoch aus ökologischer Sicht wegen der hohen Schwermetallbelastung nicht mehr zu vertreten. Weiterhin ist bekannt, daß sich substituierte Benzoesäuren durch Oxidation der entsprechenden Aldehyde mit $H_2O_2$ herstellen lassen.

In EP-A-424 242 wird in Beispiel 14 die Oxidation von 2.6-Dichlorbenzaldehyd beschrieben. Der Aldehyd wird hier in hoher Verdünnung in einem Lösungsmittel mit wäßrigem $H_2O_2$ oxidiert. Die dort angegebene Ausbeute von 54 % und die Verwendung großer Lösungsmittelmengen sind für eine technische Realisierung jedoch höchst unbefriedigend.

In US-A-4,228,302 wird in den Beispielen 6 bis 8 die Oxidation von 2,4,6-Trimethylbenzaldehyd zu der korrespondierenden Carbonsäure durch Luftsauerstoff beschrieben. Hohe Ausbeuten an Säure konnten nur dann erzielt werden, wenn der Aldehyd in Gegenwart von drei mol Equivalenten an Cyclohexanon und einem Autoxidationsinitiator durchgeführt wird. Das Cyclohexanon hat hier sowohl die Funktion eines Lösemittels als auch die eines Reduktionsmittels, so daß Caprolacton als unerwünschtes Nebenprodukt entsteht. Die dort angegebenen Reaktionszeiten von mehr als 29 Stunden sind allerdings aus ökonomischer Sicht äußerst unbefriedigend.

Der vorliegenden Erfindung lag daher die Aufgabe zugrunde, den zuvor genannten Nachteilen abzuhelfen.

Demgemäß wurde ein neues und verbessertes Verfahren zur Herstellung von o,o-disubstituierten Benzoesüuren der allgemeinen Formel I

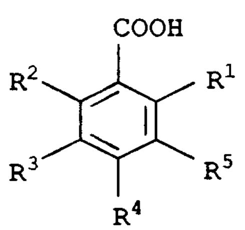

(I),

in der

| | |
|---|---|
| $R^1$,$R^2$,$R^3$,$R^4$,$R^5$ | $C_1$- bis $C_8$-Alkyl, $C_1$- bis $C_8$-Alkoxy, $C_3$- bis $C_8$-Cycloalkyl, $C_4$- bis $C_{20}$-Alkylcycloalkyl, Halogen, Aryl, $C_7$- bis $C_{20}$-Aralkyl und $C_7$- bis $C_{20}$-Alkylaryl |
| $R^3$,$R^4$,$R^5$ | zusätzlich Wasserstoff, |

bedeuten, welches dadurch gekennzeichnet ist, daß man o,o-disubstituierte Aldehyde der allgemeinen Formel II

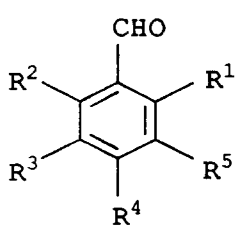

(II),

in der die Reste $R^1$, $R^2$, $R^3$, $R^4$ und $R^5$ die oben genannten Bedeutungen besitzen, durch Oxidation mit Sauerstoff oder Sauerstoff enthaltenden Gasen in Gegenwart von katalytischen Mengen eines Übergangsmetallsalzes bei Temperaturen von 50 bis 180 °C und gegebenenfalls eines Promotors umsetzt.

Das erfindungsgemäße Verfahren läßt sich wie folgt durchführen:

Man kann z.B. das Übergangsmetallsalz und gegebenenfalls den Promotor in dem o,o-disubstituierten Aldehyd auflösen und Sauerstoff oder einen Sauerstoff enthaltenden Gasstrom bei Temperaturen von 50 bis 180 °C, bevorzugt 80 bis 170 °C, besonders bevorzugt 100 bis 160 °C und Drücken von 0,01 bis 50 bar, bevorzugt 0,1 bis 5 bar, besonders bevorzugt bei Normaldruck (Atmosphärendruck) durch die Reaktionsmischung leiten. Die entstehenden o,o-disubstituierten Benzoesäuren können durch konventionelle Methoden, wie z.B. Kristallisation oder Extraktion, aus dem Reaktionsgemisch erhalten werden.

Die Umsetzung kann in Gegenwart eines inerten Lösungsmittels, bevorzugt im Wesentlichen in Abwesenheit eines Lösungsmittels durchgeführt werden. Als Lösungsmittel eignen sich Essigsäure, Propionsäure, Buttersäure, 2-Ethylenhexansäure, Methylbenzoat, Dimethyladipat, Bernsteinsäuredimethylester, 1,5,5-Trimethylpyrrolidon.

Sauerstoff dient als Oxidationsmittel; der Sauerstoff wird mindestens in stöchiometrischer Menge, bevorzugt im Uberschuß bis zu 20 mol Äquivalenten eingesetzt. Der Sauerstoff kann in reiner Form oder in Verdünnung mit einem unter den Reaktionsbedingungen inerten Gas eingesetzt werden. Solche unter den Reaktionsbedingungen inerten Gase sind z.B. Stickstoff, Kohlendioxid, Argon oder Helium oder Mischungen dieser Gase. Bevorzugtes Gas zur Verdünnung des Sauerstoffs ist Stickstoff. Besonders bevorzugt ist Luft als Oxidationsmittel. Die Konzentration an Sauerstoff in dem Sauerstoff enthaltenden Gas beeinflußt die Selektivität der Reaktion nur untergeordnet. Die Oxidationsmittel sind im Wesentlichen frei von Oxiden wie Metalloxiden z.B. Mangandioxid, Chromoxiden oder Kaliumpermanganat.

Als Übergangsmetallsalze kommen Salze vom Cobalt, Mangan oder Eisen in Frage. Vorzugsweise verwendet man im Reaktionsmedium lösliche Salze. Dies sind z.B. die Acetate, Propionate, 2-Ethylhexanoate, Stearate, Oleate, allgemein Salze von Fettsäuren, Naphthenate oder Acetylacetonate. Bevorzugt sind Cobaltacetat, Manganacetat oder Eisenacetat. Besonders bevorzugt sind Salze des Cobalts wie z.B. Cobaltacetat, Cobaltpropionat, Cobaltacetylacetonat oder Cobaltethylhexanoat. Die Menge an Übergangsmetallsalz kann in weiten Grenzen variiert werden. Bevorzugt ist ein Bereich zwischen 1 und 1000 ppm (parts per million) Übergangsmetallsalz berechnet als Metall bezogen auf die eingesetzte Menge an zu oxidierendem o,o-disubstituiertem Aldehyd II. Besonders bevorzugt ist der Bereich zwischen 5 und 100 ppm Übergangsmetallsalz berechnet als Metall bezogen auf die o,o-disubstituierten Aldehyde II.

Die katalytische Aktivität von Übergangsmetallsalzen kann vorzugsweise durch geeignete Promotoren unterstützt bzw. verstärkt werden (R.A. Sheldon, J.K. Kochi, Metal Catalyzed Oxidation of Organic Compounds, Academic Press, 1981, Seiten 315 bis 337 und dort angegebene Literaturzitate). Als Promotoren kommen aliphatische oder cycloaliphatische Aldehyde oder Ketone wie z.B. Acetaldehyd, Propionaldehyd, Isobutyraldehyd, Methylethylketon, Diethylketon, Cyclohexanon, Cylopentanon in Frage. Die Menge an Promotor kann ebenfalls über einen weiten Bereich variiert werden. Vorzugsweise verwendet man 0,001 bis 0,1 mol Promotor pro 1 mol zu oxidierender Ausgangsverbindung. Besonders bevorzugt ist ein Bereich zwischen 0,005 und 0,05 mol Promotor bezogen auf ein mol zu oxidierendem o,o-disubstituiertem Aldehyd II.

Die Substituenten R1, R2, R3, R4 und R5 in den Verbindungen I und II haben folgende Bedeutungen:

$R^1$,$R^2$,$R^3$,$R^4$,$R^5$ unabhängig voneiander

- $C_1$- bis $C_8$-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, bevorzugt $C_1$- bis $C_4$-Alkyl wie Methyl, Ethyl, n-Propyl, iso-Propyl, n-Butyl, iso-Butyl, sec.-Butyl und tert.-Butyl, besonders bevorzugt Methyl und Ethyl,
- $C_1$- bis $C_8$-Alkoxy wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.- Hexoxy, n-Heptoxy, iso-Heptoxy, n-Octoxy, iso-Octoxy, bevorzugt $C_1$- bis $C_4$-Alkoxy wie Methoxy, Ethoxy, n-Propoxy, iso-Propoxy, n-Butoxy, iso-Butoxy, sec.-Butoxy, tert.-Butoxy, besonders bevorzugt Methoxy und Ethoxy,
- $C_3$- bis $C_8$-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl, besonders bevorzugt Cyclopentyl und Cyclohexyl,
- $C_4$- bis $C_{20}$-Alkyl-cycloalkyl, bevorzugt $C_4$- bis $C_{12}$-Cycloalkyl-alkyl wie Cyclopentyl-methyl, Cyclohexylmethyl und Cycloheptylmethyl, besonders bevorzugt Cyclohexylmethyl,
- Halogen wie Fluor, Chlor, Brom und Jod, bevorzugt Fluor, Chlor und Brom, besonders bevorzugt Chlor und Brom,
- Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- $C_7$- bis $C_{20}$-Aralkyl, bevorzugt $C_7$- bis $C_{12}$-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl,

1-Phenethyl und 2-Phenethyl,
- $C_7$- bis $C_{20}$-Alkylaryl, bevorzugt $C_7$- bis $C_{12}$-Alkylphenyl wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,

$R^3$,$R^4$,$R^5$ unabhängig voneinander zusätzlich
- Wasserstoff.

Als Substitutionsmuster der Verbindungen I und II kommen bevorzugt folgende in Frage: Am aromatischen Kern in 2- und 6-Stellung disubstituierte oder in 2-, 4- und 6-Stellung bzw. 2-, 5- und 6-Stellung trisubstituierte Verbindungen I und II. Besonders bevorzugt sind die in 2- und 6-Stellung disubstituierten bzw in 2-, 4- und 6-Stellung trisubstituierten Verbindungen I und II, worin die Substituenten gleich oder verschieden sind und Methyl, Ethyl, Propyl, Methoxy, Ethoxy oder Chlor oder Brom bedeuten.

Bevorzugte Verbindungen I und II sind:

| o,o-disubstituierte Benzoesäuren | o,o-disubstituierte Aldehyde |
|---|---|
| 2,6-Dimethylbenzoesäure | 2,6-Dimethylbenzaldehyd |
| 2,6-Diethylbenzoesäure | 2,6-Diethylbenzaldehyd |
| 2,6-Dimethoxybenzoesäure | 2,6-Dimethoxybenzaldehyd |
| 2,6-Diethoxybenzoesäure | 2,6-Diethoxybenzaldehyd |
| 2,6-Dichlorbenzoesäure | 2,6-Dichlorbenzaldehyd |
| 2,6-Dibrombenzoesäure | 2,6-Dibrombenzaldehyd |
| 2,4,6-Trichlorbenzoesäure | 2,4,6-Trichlorbenzaldehyd |
| 2,4,6-Tribrombenzoesäure | 2,4,6-Tribrombenzaldehyd |
| 2,4,6-Trimethylbenzoesäure | 2,4,6-Trimethylbenzaldehyd |
| 2,4,6-Triethylbenzoesäure | 2,4,6-Triethylbenzaldehyd |
| 2,4,5,6-Tetramethylbenzoesäure | 2,4,5,6-Tetramethylbenzaldehyd |
| 2,6-Dimethyl-4-phenylbenzoesäure | 2,6-Dimethyl-4-phenylbenzaldehyd |

Die nach dem erfindungsgemäßen Verfahren herstellbaren Benzoesäuren der allgemeinen Formel I sind wertvolle Ausgangsstoffe für Farbstoffe, Schädlingsbekämpfungsmittel, Pharmazeutika und Photoinitiatoren. Beispielsweise ergeben die den Benzoesäuren der allgemeinen Formel I entsprechenden Benzoylchloride durch Umsetzung mit Alkoxyphosphinen Verbindungen, die als wertvolle Photoinitiatoren verwendet werden können. Weitere Verwendungen sind in den vorgenannten Veröffentlichungen und Ullmanns Enzyklopädie der technischen Chemie, Band 4, Seiten 272 bis 291, enthalten.

## Beispiele

### Beispiel 1

Eine Mischung aus 300 g 2,4,6-Trimethylbenzaldehyd, 25 mg Cobalt(II)acetat * 4 $H_2O$ und 2,5 g Methylethylketon wird auf 100 °C aufgeheizt. Anschließend leitet man über 4 Stunden einen Luftstrom durch die Mischung, wobei ein Umsatz von ca. 30 % an eingesetztem Aldehyd erreicht wird, was durch Titration der entstehenden Säure ermittelt wird. Hiernach läßt man auf Raumtemperatur abkühlen und verdünnt die Mischung mit Methyltertiärbutylether. Die 2,4,6-Trimethylbenzoesäure wird mit einer wäßrigen, alkalischen Lösung extrahiert und anschließend durch Ansäuern aus dem wäßrigen Medium ausgefällt und abfiltriert. Ausbeute an Trimethylbenzoesäure 86 g (86 % der Theorie) bezogen auf umgesetzten Aldehyd. Die Mutterlauge, die nicht umgesetzten Aldehyd enthält, kann nach dem Entfernen des Ethers ohne weitere Behandlung wieder eingesetzt werden.

Die Verfahrensweise in den Beispielen 2 bis 8 ist analog Beispiel 1, wenn nicht anders beschrieben. Der in Beispiel 1 verwendete Promotor wurde ebenfalls in den Beispielen 2 bis 7 in der gleichen Menge eingesetzt. In Beispiel 8 (Vergleichsbeispiel) wurde auf das Übergangsmetallsalz und den Promotor verzichtet. Die Versuche wurden alle nach 4 Stunden abgebrochen. Die verwendeten Aldehyde, Reaktionsbedingungen, eingesetzte Mengen und die entstehenden Benzoesäuren sind in Tabelle 1 angegeben:

Tabelle 1

| Bsp. Nr. | Aldehyd (Menge) | Katalysator (Menge) | Temp. (°C) | Umsatz Aldehyd | Benzoesäure (Ausbeute)[8] |
|---|---|---|---|---|---|
| 2 | TMBA[1] (300g) | CoAcetat[2] (25 mg) | 150 | 34% | TMBS[3] (91 %) |
| 3 | TMBA (300g) | CoAcetat (100 mg) | 150 | 36% | TMBS (89%) |
| 4 | TMBA (300g) | MnAcetat[4] (30 mg) | 150 | 27% | TMBS (76%) |
| 5 | TMBA (300g) | MnAcetat (100 mg) | 150 | 38% | TMBS (78%) |
| 6 | DCBA[5] (300g) | CoAcetat (30 mg) | 130 | 24% | DCBS[6] (73%) |
| 7 | DCBA (300g) | MnAcetat (40 mg) | 130 | 21% | DCBS (64%) |
| 8[7] | TMBA (300g) | ------- | 150 | 2% | TMBS (--) |

Erläuterungen zu Tabelle 1:

[1] TMBA = 2,4,6-Trimethylbenzaldehyd

[2] CoAcetat = Kobalt(II)acetat * 4 $H_2O$

[3] TMBS = 2,4,6-Trimethylbenzoesäure

[4] MnAcetat = Mangan(II)acetat * 4 $H_2O$

[5] DCBA = 2,6-Dichlorbenzaldehyd

[6] DCBS = 2,6-Dichlorbenzoesäure

[7] Vergleichsbeispiel : Unter den gegebenen Reaktionsbedingungen wird keine Benzoesäure gebildet

[8] Die in den Beispielen 2 bis 8 angegebenen Ausbeuten beziehen sich auf umgesetzten Aldehyd.

## Patentansprüche

**1.** Verfahren zur Herstellung von o,o-disubstituierten Benzoesäuren der allgemeinen Formel I

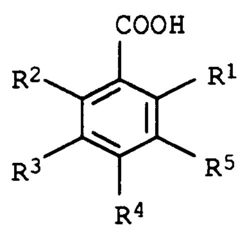

(I),

in der

$R^1,R^2,R^3,R^4,R^5$ $C_1$- bis $C_8$-Alkyl, $C_1$- bis $C_8$-Alkoxy, $C_3$- bis $C_8$-Cycloalkyl, $C_4$- bis $C_{20}$-Alkylcycloalkyl, Halogen, Aryl, $C_7$- bis $C_{20}$-Aralkyl und $C_7$- bis $C_{20}$-Alkylaryl

$R^3,R^4,R^5$ zusätzlich Wasserstoff,

bedeuten, dadurch gekennzeichnet, daß man o,o-disubstituierte Aldehyde der allgemeinen Formel II

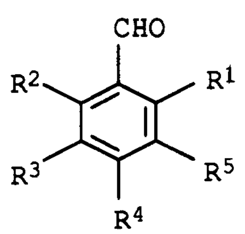

(II),

in der die Reste $R^1$, $R^2$, $R^3$, $R^4$ und $R^5$ die oben genannten Bedeutungen besitzen, durch Oxidation mit Sauerstoff oder Sauerstoff enthaltenden Gasen in Gegenwart von katalytischen Mengen eines Übergangsmetallsalzes bei Temperaturen von 50 bis 180°C und gegebenenfalls eines Promotors umsetzt.

**2.** Verfahren zur Herstellung von o,o-disubstituierten Benzoesäuren nach Anspruch 1, dadurch gekennzeichnet, daß man als Sauerstoff enthaltendes Gas Luft verwendet.

3. Verfahren zur Herstellung von o,o-disubstituierten Benzoesäuren nach Anspruch 1, dadurch gekennzeichnet, daß man als Übergangsmetall eines der Salze des Kobalts, Eisens oder Mangans verwendet.

4. Verfahren zur Herstellung von o,o-disubstituierten Benzoesäuren nach Anspruch 1, dadurch gekennzeichnet, daß man als Übergangsmetallsalz Cobalt-(II)-acetat verwendet.

5. Verfahren zur Herstellung von o,o-disubstituierten Benzoesäuren nach Anspruch 1, dadurch gekennzeichnet, daß $R^1$ und $R^2$ Chlor und $R^3$, $R^4$ und $R^5$ Wasserstoff bedeuten.

6. Verfahren zur Herstellung von o,o-disubstituierten Benzoesäuren nach Anspruch 1, dadurch gekennzeichnet, daß $R^1$, $R^2$ und $R^4$ Methyl und $R^3$ und $R^5$ Wasserstoff bedeuten.

7. Verfahren zur Herstellung von o,o-disubstituierten Benzoesäuren nach Anspruch 1, dadurch gekennzeichnet, daß man die Oxidation bei einer Temperatur von 100 bis 160 °C durchführt.